# EUROPEAN PATENT SPECIFICATION

(11) **EP 1 617 757 B1**
(45) Date of publication and mention of the grant of the patent: **26.08.2009**
(21) Application number: 04719393.3
(22) Date of filing: 11.03.2004
(51) Int. Cl.: A61B 5/00, A61B 10/00, G02B 1/04, A61F 9/00

(54) **DEVICES FOR COLLECTING ANALYTES OF INTEREST IN TEARS**
VORRICHTUNGEN ZUM AUFFANGEN VON INTERESSIERENDEN ANALYTEN IN TRÄNEN
DISPOSITIFS PERMETTANT DE RECUEILLIR DES ANALYSATS EN RAPPORT AVEC LES LARMES

(30) Priority: 12.03.2003 US 454150 P
(43) Date of publication of application: 25.01.2006
(73) Proprietor: NOVARTIS AG, 4056 Basel (CH)
(72) Inventor: CARNEY, Fiona, Patricia, Atlanta, GA 30329 (US); MORRIS, Carol, Ann, Duluth, GA 30097 (US); QIU, Yongxing, Duluth, GA 30097 (US)
(74) Representative: Bohest AG
(86) International application number: PCT/EP2004/002543
(87) International publication number: WO 2004/080297

(56) References cited:
- DE-C- 3 218 999
- US-A- 4 526 178
- US-A- 5 869 231
- US-A1- 2002 192 657
- US-A1- 2003 045 783
- US-B1- 6 451 871

## Description

The invention is related to a device for collecting one or more analytes of interest in tears. In particular, the invention is related to a contact lens capable of collecting one or more analytes of interest in tears. In addition, the invention provides methods and kits for collecting and/or assaying of analytes of interest in tears.

### BACKGROUND OF THE INVENTION

It is important to detect/measure one or more particular hormones in serum, for a variety of reasons, such as, for example, for assisting In diagnosing the occurrence of an endocrinological disorder, for monitoring the amount of hormones required in hormonal replacement therapy, or for assessing ovulation, pregnancy, contraception, menopause or sexual dysfunction of an individual. Historically, blood collection was required to gather information on the physiological properties of the body, including monitoring women's reproductive cycle. Blood collection is an invasive technique requiring arterial or venous puncture. A patient has to endure discomfort associated with needles or other devices to obtain blood samples for testing. In addition, blood collection sometimes can be associated with problems in various ethnic settings. Therefore, assays of serum hormones are preferably avoided or replaced by alternative non-invasive assays.

In the last decade, considerable attention was paid to substitute assays of serum analytes of interest with assays of urinary analytes of interest. For example, a number of patents and patent applications disclose non-invasive home use fertility tests based on urine analysis (see, for example, US 6,399,398; EP0236023A2; EP0656118B1; EP0703454B1; EP0745853B1; EP0745854B1; EP0728310B1). Those fertility tests are largely based on a series of concentration measurements of urinary estradiol metabolites (e.g., estrone-3-glucuronide, estradiol-3-glucuronide, estradiol-17-glucuronide, estriol-3-glucuronide, estriol-16-glucuronide), urinary luteinising hormone (LH), and/or urinary pregnanediol-3-glucuronide (P3G) (i.e., a progesterone metabolite). To be useful, such concentration data must be determined accurately, usually from a series of samples. For example, a sample may need to be collected daily over an extended sequence of days, and successive daily analyte concentrations are compared to identify a significant concentration change indicative of a change in fertility (i.e., fecundity) status. However, the volume of a body fluid (e.g., urine) produced and/or secreted under various physiological and environmental conditions can fluctuate significantly, so that the apparent concentration of the analyte may not be a true reflection of the amount of analyte being produced by the body at that time (i.e., may not be correlated with the serum concentration of the analyte under analyzed). Such variation in concentration in a body fluid of an analyte, derived from variation in the volume of a body fluid (e.g., urine) which is produced and/or secreted under different physiological and environmental conditions, is referred to hereinafter "biological concentration variability". Biological concentration variability can interfere with the comparability of such urinary concentration data. The sample to be assayed is collected while urine is being excreted. When the collected sample is analyzed for the presence of a specific analyte, such as an estradiol metabolite, the degree of fluid intake, and kidney function, has a very significant influence on the actual volume and frequency of urine excretion and consequently the concentration of the analyte. If fluid intake has been relatively high, or relatively low, during the previous few hours, the measurable concentration of analyte in the collected sample can be much lower (or higher) than normal, leading to Inaccurate and possibly misleading information. Additionally, diurnal hormone variations are affected by aging, sleep loss, night or shift work, physical exercise, jet lag, affective disorders and endocrine diseases. To overcome the disadvantages associated with currrently available methods for collecting and assaying analytes of interest in a blood and urine sample, it is desirable to identify another component of the body fluid source which can be analyzed readily and which can be correlated with the blood analysis. Tear fluid can be an alternative source for analysis of trace constituents (analytes) in a body fluid. The precorneal tear film is a thin, liquid film that covers the cornea and conjunctival epithelium. This tear film consists of hundreds of proteins and/or enzymes, which are principally originated from the active secretion by lacrimal glands, by the leakage from the plasma either across the blood/tear barrier or by leakage from tissue interstitial fluid. Analyzing plasma constituents from tears has been previously proposed (see, for example, EP0236023A2; US App. No.2002/0049389A1; U.S. Pat No. 5,352,411; and US App. No. 2001/0034500 A1). Although tear fluid is an alternative body fluid which can be analyzed, it is generally difficult or impossible to obtain a large enough tear sample to allow measurement or detection of constituents. To obtain such a volume of tears for research or analysis, investigators have generally been required to use artificial stimulation of tear production, for example, with tear-inducing chemicals, fans, and the like. Generally, a tear fluid is collected by using a capilary glass tube. However, tear collection by capillary glass tubes may be invasive and irritating and poses a risk if not carefully done. Such tear collection has to be performed by well trained professionals and is unsuitable for home use.

Moreover, the concentration of a constituent in a tear fluid could be so low that the measurement or detection of the constituent could be very difficult or inaccurate. In addition, there might exist biological concentration variability in tear fluid, depending on the method for collecting tears. For example, the concentration of some contituents in tears is flow-dependent and therefore depends on the method of collection of the tears.

Therefore, there is a need for an alternative tear collection device which can be a safer, much faster, and less irritating. There is also a need for a method of assaying an analyte of interest in a way that biological concentration variability can be minimized. A contact lens for collecting an analyte of interest in a tear fluid is known from US-A-2003/045783.
One object of the invention is to provide a user-friendly tear collection device which is capable of enriching one or more selected analytes of interest.

Another object of the invention is to provide a method for collecting selectively one or more trace constituents (analytes) in a body fluid.

A still another object of the invention is to provide a method and kits for assaying one or more analytes of interest in a tear fluid. Such method and kits have relatively high sensitivity and reliability and are suitable for patients to carry out assays in a more convenient and discreet manner (e.g., at home).

### SUMMARY OF THE INVENTION

This invention is based largely on the discovery that a contact lens, preferably a daily disposable contact lens, can be used to collect one or more analytes of interest in tear fluid, and in turn, determine the physiological state or health of a subject. The contact lens may be in its native form or may be modified to selectively enhance adsorption of one or more analytes of interest. By wearing a contact lens capable of binding one or more analytes of interest, over a period of time, for example, 15 minutes or longer, preferably one hour or longer, more preferably 2 hours or longer, even more preferably 4 hours or longer, most preferably 8 hours or longer, the one or more analytes of interest can be enriched over the period of wearing time, since the tear fluid in a normal human eye is continuously replenished. By using a contact lens capable of binding an analyte of interest in a tear fluid, one can determine the concentration of an analyte of interest accumulated over a period of time and therefore the effects of biological concentration variability on the determined concentration of the one or more analytes of interest can be minimized. Therefore, the accuracy of assays for the analytes in a body fluid can be greatly enhanced. The invention, in one aspect, provides a contact lens, preferably a hydrogel soft contact lens, more preferably a daily disposable hydrogel soft contact lens, for collecting an analyte of interest in a tear fluid. A contact lens of the invention has molecular imprints for the analyte of interest.

The invention, in another aspect, provides a method for collecting an analyte of interest in a tear fluid as defined in claim 9.

The invention, in still another aspect, provides a method for assaying an analyte of interest in a tear fluid as defined in claim 10.

The invention, in a further aspect, provides a kit for collecting an analyte of interest in a tear fluid as defined in claim 11.

The invention, in a further aspect, provides a kit for assaying an analyte of interest in a tear fluid as defined in claim 12.

### DETAILED DESCRIPTION OF PREFERRED EMBODIMENTS

Reference now will be made in detail to the embodiments of the invention, one or more examples of which are set forth below. Each example is provided by way of explanation of the invention, and is not a limitation of the invention. In fact, it will be apparent to those skilled in the art that various modifications and variations can be made in the present invention without departing from the scope of the invention. For instance, features illustrated or described as part of one embodiment, can be used on another embodiment to yield a still further embodiment. Thus, it is intended that the present invention covers such modifications and variations as come within the scope of the appended claims and their equivalents. Other objects, features and aspects of the present invention are disclosed in or are obvious from the following detailed description. It is to be understood by one of ordinary skill in the art that the present discussion is a description of exemplary embodiments only, and is not intended as limiting the broader aspects of the present invention.

Unless defined otherwise, all technical and scientific terms used herein have the same meaning as commonly understood by one of ordinary skill in the art to which this invention belongs. Generally, the nomenclature used herein and the laboratory procedures are well known and commonly employed in the art. Conventional methods are used for these procedures, such as those provided in the art and various general references. Where a term is provided in the singular, the inventors also contemplate the plural of that term. As employed throughout the disclosure, the following terms, unless otherwise indicated, shall be understood to have the following meanings.

The term "analyte" refers to a substance being tested. Exemplary analytes of interest include, but are not limited to, electrolytes and small molecules (*e*.*g*., sodium, potassium, chloride, phenylalanine, uric acid, galactose, glucose, cysteine, homocysteine, calcium, ethanol, acetylcholine and acetylcholine analogs, ornithine, blood urea nitrogen, creatinine), metallic elements (*e*.*g*., iron, copper, magnesium), polypeptide hormones (*e*.*g*., thyroid stimulating hormone, growth hormone, insulin, luteinizing hormones, chorionogonadotrophic hormone, obesity hormones such as leptin, serotonin and the like), chronically administered medications (*e*.*g*., dilantin, phenobarbital, propranolol), acutely administered medications (*e*.*g*., cocaine, heroin, ketamine), small molecule hormones (*e*.*g*., thyroid hormones, ACTH, estrogen, cortisol, progesterone and other metabolic steroids), markers of inflammation and/or allergy (*e*.*g*., histamine, IgE, cytokines), lipids (*e.g*., cholesterol, apolipo protein A₁), proteins and enzymes (*e*.*g*., lactoferrin, lysozyme, tear-specific prealbumin or lipocalin, albumin, complement, coagulation factors, liver function enzymes, heart damage enzymes, ferritin), markers of infection (*e*.*g*., virus components, immunoglobulins such as IgM, IgG, etc., proteases, protease inhibitors), and/or metabolites (*e*.*g*., lactate, ketone bodies). The term "contact lens" employed herein in a broad sense and is intended to encompass any hard or soft lens used on the eye or ocular vicinity for vision correction, diagnosis, sample collection, drug delivery, wound healing, cosmetic appearance (*e*.*g*., eye color modification), or other ophthalmic applications.

"Ophthalmically compatible", as used herein, refers to a material or surface of a material which may be in intimate contact with the ocular environment for an extended period of time without significantly damaging the ocular environment and without significant user discomfort. Thus, an ophthalmically compatible contact lens will not produce significant corneal swelling, will adequately move on the eye with blinking to promote adequate tear exchange, will not have substantial amounts of protein or lipid adsorption, and will not cause substantial wearer discomfort during the prescribed period of wear.

"Ocular environment", as used herein, refers to ocular fluids (e.g., tear fluid) and ocular tissue (e.g., the cornea) and/or conjunctiva which may come into intimate contact with a contact lens.

A "hydrogel material" refers to a polymeric material which can absorb at least 10 percent by weight of water when it is fully hydrated. Generally, a hydrogel material is obtained by polymerization or copolymerization of at least one hydrophilic monomer in the presence of or in the absence of additional monomers and/or macromers. Exemplary hydrogels include, but are not limited to, poly(vinyl alcohol) (PVA), modified polyvinylalcohol (e.g., as nelfilcon A), poly(hydroxyethyl methacrylate), poly(vinyl pyrrolidone), PVAs with polycarboxylic acids (e.g., carbopol), polyethylene glycol, polyacrylamide, polymethacrylamide, silicone-containing hydrogels, polyurethane, polyurea, and the like. A hydrogel can be prepared according to any methods known to a person skilled in the art.

A "monomer" means a low molecular weight compound that can be polymerized. Low molecular weight typically means average molecular weights less than 700 Daltons.
A "hydrophilic vinylic monomer" refers to a monomer which as a homopolymer typically yields a polymer that is water-soluble or can absorb at least 10 percent by weight water.
A "macromer" refers to medium and high molecular weight compounds or polymers that contain functional groups capable of further polymerization. Medium and high molecular weight typically means average molecular weights greater than 700 Daltons.

"Polymer" means a material formed by polymerizing one or more monomers.

"Surface modification", as used herein, means that an article has been treated in a surface treatment process (or a surface modification process), in which, by means of contact with a vapor or liquid, and/or by means of application of an energy source (1) a coating is applied to the surface of an article, (2) chemical species are adsorbed onto the surface of an article, (3) the chemical nature (e.g., electrostatic charge) of chemical groups on the surface of an article are altered, or (4) the surface properties of an article are otherwise modified.

Exemplary surface treatment processes include, but are not limited to, a surface treatment by energy (e.g., a plasma, a static electrical charge, irradiation, or other energy source), chemical treatments, the grafting of hydrophilic monomers or macromers onto the surface of an article, and layer-by-layer deposition of polyelectrolytes. A preferred class of surface treatment processes are plasma processes, in which an ionized gas is applied to the surface of an article. Plasma gases and processing conditions are described more fully in U.S. Pat. Nos. 4,312,575 and 4,632,844. The plasma gas is preferably a mixture of lower alkanes and nitrogen, oxygen or an inert gas.

"LbL coating", as used herein, refers to a coating that is not covalently attached to a contact lens and is obtained through a laypr-by-layer ("LbL") deposition of polyionic or charged materials on an article.

The term "bilayer" is employed herein in a broad sense and is intended to encompass: an LbL coating structure formed on a contact lens by alternatively applying, in no particular order, one layer of a first polyionic material (or charged material) and subsequently one layer of a second polyionic material (or charged material) having charges opposite of the charges of the first polyionic material (or the charged material); or a coating structure formed onto a contact lens by alternatively applying, in no particular order, one layer of a first charged polymeric material and one layer of a non-charged polymeric material or a second charged polymeric material. It should be understood that the layers of the first and second coating materials (described above) may be intertwined with each other in the bilayer.

A medical device having a core material and an LbL coating, which comprises at least one layer of a charged polymeric material and one layer of a non-charged polymeric material that can be non-covalently bonded to the charged polymeric material, can be prepared according to a method disclosed, for example, in EP-A-1046068. As used herein, "asymmetrical coatings" on a contact lens refers to the different coatings on the first surface and the opposite second surface of the contact lens. As used herein, "different coatings" refers to two coatings that have different surface properties or functionalities.

An "innermost layer", as used herein, refers to the first layer of an LbL coating, which is applied onto the surface of a contact lens.

A "capping layer", as used herein, refers to the last layer of a coating material which is applied onto the surface of a contact lens.

A "polyquat", as used herein, refers to a polymeric quaternary ammonium group-containing compound.

A "charged polymeric material" or a "polyionic material" refers to a charged polymer that has a plurality of charged groups in a solution, or a mixture of charged polymers each of which has a plurality of charged groups in a solution. Exemplary charged polymers includes polyelectrolytes, p- and n-type doped conducting polymers. Charged polymeric materials include both polycationic (having positive charges) and polyanionic (having negative charges) polymeric materials.

As used herein, "increased adsorption of an analyte" in reference to a contact lens having surface charges means that the contact lens with surfaces charges can bind a higher amount of the analyte of interest compared with a contact lens made of similar material and essentially free of surface charges. The contact lens with surface charges can bind the analyte of interest in an amount which is preferably at least about 25%, more preferably at least about 50%, even more preferably at least about 75% higher than the amount of the analyte of interest bound by a contact lens made of similar material and essentially free of surface charges

The term "receptor" is employed herein in a broad sense and is intended to encompass, for example, a protein or fragment thereof or a chemical compound that is capable of binding said analyte in a sample. Exemplary receptors include, without limitation, antibodies or fragments thereof, lectins or fragments thereof, hormone receptors or fragments thereof, drug receptors or fragment thereof, enzymes or fragment thereof, aptamers, nucleic acids, nucleic acid analogs, and the like.

The invention, in one aspect, provides a contact lens for collecting an analyte of interest in a tear fluid, wherein the contact lens is capable of binding the analyte of interest.
A contact lens of the invention is preferably a soft contact lens, more preferably a hydrogel soft contact lens, even more preferably a daily disposable hydrogel soft contact lens (for example, DAILIES® lenses).

In accordance with one preferred embodiment of the invention, a contact lens of the invention comprises surface charges present in a density sufficient to impart to the contact lens an increased adsorption of the analyte of interest.

Surface charges, either positive charges or negative charges, can be introduced on the surface of a contact lens by preparing the contact lens from a composition comprising a positively or negatively charged monomer or macromer. Any known suitable charged monomers or macromers can be used in the preparation of a contact lens of the invention. Surface charges can also be introduced on the surface of a contact lens by altering the chemical nature (i.e., electrostatic charge) of chemical groups on its surface, for example, by means of contact with a vapor or liquid, and/or by means of application of an energy source (e.g., an plasma treatment, an electron beam treatment, a corona discharge, or an X-ray treatment).

Surface charges are preferably introduced on the surface of a contact lens by applying an LbL coating composed of at least one layer of a polyionic material. Application of an LbL coating may be accomplished in a number of ways as described in US Patent No. 6,451,871 or US-A-2001-0045676 or EP-A-1,252222. One coating process embodiment involves solely dip-coating and dip-rinsing steps. Another coating process embodiment involves solely spray-coating and spray-rinsing steps. However, a number of alternatives involve various combinations of spray- and dip-coating and rinsing steps may be designed by a person having ordinary skill in the art.

For example, a solely dip-coating process involves the steps of: (a) immersing a medical device in a first coating solution of a first polyionic material; (b) optionally rinsing the medical device by immersing the medical device in a first rinsing solution; (c) immersing said medical device in a second coating solution of a second polyionic material to form a first polyelectrolyte bilayer of the first and second polyionic materials, wherein the second polyionic material has charges opposite of the charges of the first polyionic material; (d) optionally rinsing said medical device by immersing the medical device in the rinsing solution; and (e) optionally repeating steps (a) to (d) for a number of times to form additional polyelectrolyte bilayers. A thicker LbL coating can be produced by repeating steps (a) to (d) preferably for 2 to 40 times. A preferred number of bilayers is about 5 to about 20 bilayers.

While more than 20 bilayers are possible, it has been found that delamination may occur in some LbL coatings having an excessive number of bilayers.

The immersion time for each of the coating and rinsing steps may vary depending on a number of factors. Preferably, immersion of the core material into the polyionic solution occurs over a period of about 1 to 30 minutes, more preferably about 2 to 20 minutes, and most preferably about 1 to 5 minutes. Rinsing may be accomplished in one step, but a plurality of rinsing steps can be quite efficient.

Another embodiment of the coating process is a single dip-coating process as described in EP-A-1252222. Such single dip-coating process involves dipping a core material of a medical device in a solution containing a negatively charged polyionic material and a positively charged polyionic material in an amount such that the molar charge ratio of said solution is from about 3:1 to about 100:1. Multiple bilayers can be formed on a medical device by using this single dip-coating process.

Another embodiment of the coating process involves a series of spray coating techniques. For example, a solely spray-coating process generally includes the steps of: (a) spraying a medical device with a first coating solution of a first polyionic material; (b) optionally rinsing the medical device by spraying it with a rinsing solution; (c) spraying said medical device with a second coating solution of a second polyionic material to form a first polyelectrolyte bilayer of the first and second polyionic materials, wherein the second polyionic material has charges opposite of the charges of the first polyionic material; (d) optionally rinsing said medical device by spraying it with the rinsing solution; (e) optionally repeating steps (a) to (d) for a number of times. A thicker LbL coating can be produced by repeating steps (a) to (d) preferably for 2 to 40 times.

The spray coating application may be accomplished via a process selected from the group consisting of an air-assisted atomization and dispensing process, an ultrasonic-assisted atomization and dispensing process, a piezoelectric assisted atomization and dispensing process, an electro-mechanical jet printing process, a piezo-electric jet printing process, a piezo-electric with hydrostatic pressure jet printing process, and a thermal jet printing process; and a computer system capable of controlling the positioning of the dispensing head of the spraying device on the ophthalmic lens and dispensing the coating liquid. Those spraying coating processes are described for example in EP-A-1262307. By using such spraying coating processes, an asymmetrical coating can be applied to a medical device. For example, the back surface of a contact lens can be coated with a hydrophilic and/or lubricous coating material and the front surface of the contact lens can be coated with an antimicrobial material. It is also possible to produce a coating on a contact lens, the coating having a functional pattern so as to provide simultaneously multiple benefits to a wearer. The polyionic materials that may be employed in the present invention include polyanionic and polycationic polymers. Examples of suitable polyanionic polymers include, for example, a synthetic polymer, a biopolymer or modified biopolymer comprising carboxy, sulfo, sulfato, phosphono or phosphato groups or a mixture thereof, or a salt thereof, for example, a biomedical acceptable salt and especially an ophthalmically acceptable salt thereof when the article to be coated is an ophthalmic device.

Examples of synthetic polyanionic polymers are: a linear polyacrylic acid (PAA), a branched polyacrylic acid, a polymethacrylic acid (PMA), a polyacrylic acid or polymethacrylic acid copolymer, a maleic or fumaric acid copolymer, a poly(styrenesulfonic acid) (PSS), a polyamido acid, a carboxy-terminated polymer of a diamine and a di- or polycarboxylic acid (e.g., carboxy-terminated Starburst^{™} PAMAM dendrimers from Aldrich), a poly(2-acrylamido-2-methylpropanesulfonic acid) (poly-(AMPS)), an alkylene polyphosphate, an alkylene polyphosphonate, a carbohydrate polyphosphate or carbohydrate polyphosphonate (e.g., a teichoic acid). Examples of a branched polyacrylic acid include a Carbophil^{®} or Carbopol^{®} type from Goodrich Corp. Examples of a copolymer of acrylic or methacrylic acid include a copolymerization product of an acrylic or methacrylic acid with a vinyl monomer including, for example, acrylamide, N,N-dlmethyl acrylamide or N-vinylpyrrolidone. Examples of polyanionic biopolymers or modified biopolymers are: hyaluronic acid, glycosaminoglycane such as heparin or chondroitin sulfate, fucoidan, poly-aspartic acid, poly-glutamic acid, carboxymethyl cellulose, carboxymethyl dextrans, alginates, pectins, gellan, carboxyalkyl chitins, carboxymethyl chitosans, sulfated polysaccharides.

A preferred polyanionic polymer is a linear or branched polyacrylic acid or an acrylic acid copolymer. A more preferred anionic polymer is a linear or branched polyacrylic acid. A branched polyacrylic acid in this context is to be understood as meaning a polyacrylic acid obtainable by polymerizing acrylic acid in the presence of suitable (minor) amounts of a di- or polyvinyl compound.

A suitable polycationic polymer as part of the bilayer is, for example, a synthetic polymer, biopolymer or modified biopolymer comprising primary, secondary or tertiary amino groups or a suitable salt thereof, preferably an ophthalmically acceptable salt thereof, for example a hydrohalogenide such as a hydrochloride thereof, in the backbone or as substituents. Polycationic polymers comprising primary or secondary amino groups or a salt thereof are preferred.

Examples of synthetic polycationic polymers are:
(i) a polyallylamine (PAH) homo- or copolymer, optionally comprising modifier units;
(ii) a polyethyleneimine (PEI);
(iii) a polyvinylamine homo- or copolymer, optionally comprising modifier units;
(iv) a poly(vinylbenzyl-tri-C₁-C₄-alkylammonium salt), for example a poly(vinylbenzyl-trimethyl ammoniumchloride);
(v) a polymer of an aliphatic or araliphatic dihalide and an aliphatic N,N,N',N'-tetra-C₁-C₄-alkyl-alkylenediamine, for example a polymer of (a) propylene-1,3-dichloride or -dibromide or p-xylylene dichloride or dibromide and (b) N,N,N',N'-tetramethyl-1,4-tetramethylene diamine;
(vi) a poly(vinylpyridine) or poly(vinylpyridinium salt) homo- or copolymer;
(vii) a poly(N,N-diallyl-N,N-di-C₁-C₄-alkyl-ammoniumhalide);
(viii) a homo- or copolymer of a quaternized di-C₁-C₄-alkyl-aminoethyl acrylate or methacrylate, for example a poly(2-hydroxy-3-methacryloylpropyltri-C₁-C₂-alkylammonium salt) homopolymer such as a a poly(2-hydroxy-3-methacryloylpropyltri-methylammonium chloride), or a quaternized poly(2-dimethylaminoethyl methacrylate or a quaternized poly(vinylpyrrolidone-co-2-dimethylaminoethyl methacrylate);
(ix) polyquat; or
(x) a polyaminoamide (PAMAM), for example a linear PAMAM or a PAMAM dendrimer such as an amino-terminated Starbust^{™} PAMAM dendrimer (Aldrich).

The above mentioned polymers comprise in each case the free amine, a suitable salt thereof, for example a biomedically acceptable salt or in particular an ophthalmically acceptable salt thereof, as well as any quaternized form, if not specified otherwise.

Suitable comonomers optionally incorporated in the polymers according to (i), (iii), (vi) or (viii) above are, for example, hydrophilic monomers such as acrylamide, methacrylamide, N,N-dimethyl acrylamide, N-vinylpyrrolidone and the like.

Examples of polycationic biopolymers or modified biopolymers that may be employed in the bilayer of the present invention include: basic peptides, proteins or glycoproteins, for example, a poly-ε-lysine, albumin or collagen, aminoalkylated polysaccharides such as a chitosan or aminodextrans.

Particular polycationic polymers for forming the bilayer of the present invention include a polyallylamine homopolymer, a polyallylamine comprising modifier units of the above formula (II); a polyvinylamine homo- or -copolymer or a polyethyleneimine homopolymer, in particular a polyallylamine or polyethyleneimine homopolymer, or a poly(vinylamine-co-acrylamide) copolymer.

The foregoing lists are intended to be exemplary, but clearly are not exhaustive. A person skilled in the art, given the disclosure and teaching herein, would be able to select a number of other useful polyionic materials.

The density of surface charges of a contact lens can be determined according to any known suitable method. Preferably, a contact lens of the invention has a surface charge density at which the contact lens can bind the analyte of interest in an amount which is at least about 25% higher than the amount of the analyte of interest bound by a contact lens made of similar material and essentially free of surface charges.

In accordance with another preferred embodiment of the invention, a contact lens of the invention comprises a coating comprising a receptor which binds specifically the analyte of interest.

A receptor-containing coating can cover whole or part of the surface of a contact lens. A receptor-containing coating on a contact lens can be a layer of a receptor which is covalently attached to the contact lens. Such contact lens can be prepared by first functionalizing the surface of a preformed contact lens to obtain function groups and then covalently attaching a layer of receptor. Surface modification (or functionalization) of a medical device, for example, a contact lens, is well known to a person skilled in the art. Any known suitable method can be used.

For example, the surface modification of a contact lens includes, without limitation, the grafting of monomers or macromers onto polymers to make the lens biocompatible, wherein monomers or macromers contain functional groups, for example, such as hydroxyl group, amine group, amide group, sulfhydryl group, -COOR (R and R' are hydrogen or C₁ to C₈ alkyl groups), halide (chloride, bromide, iodide), acyl chloride, isothiocyanate, isocyanate, monochlorotriazine, dichlorotriazine, mono- or di-halogen substituted pyridine, mono- or di-halogen substituted diazine, phosphoramidite, maleimide, aziridine, sulfonyl halide, hydroxysuccinimide ester, hydroxysulfosuccinimide ester, imido ester, hydrazine, axidonitrophenyl group, azide, 3-(2-pyridyl dithio)proprionamide, glyoxal, aldehyde, and epoxy.

It is well known in the art that a pair of matching functional groups can form a covalent bond or linkage under known reaction conditions, such as, oxidation-reduction conditions, dehydration condensatipn conditions, addition conditions, substitution (or displacement) conditions, 2+2 cyclo-addition conditions, Diels-Alder reaction conditions, ROMP (Ring Opening Metathesis Polymerization) conditions, vulcanization conditions, cationic crosslinking conditions, and epoxy hardening conditions. For example, an amino group is covalently bondable with aldehyde (Schiff base which is formed from aldehyde group and amino group may further be reduced); an hydroxyl group and an amino group are covalently bondable with carboxyl group; carboxyl group and a sulfo group are covalently bondable with hydroxyl group; a mercapto group is covalently bondable with amino group; or a carbon-carbon double bond is covalently bondable with another carbon-carbon double bond. Exemplary covalent bonds or linkage, which are formed between pairs of crosslinkable groups, include without limitation, ester, ether, acetal, ketal, vinyl ether, carbamate, urea, amine, amide, enamine, imine, oxime, amidine, iminoester, carbonate, orthoester, phosphonate, phosphinate, sulfonate, sulfinate, sulfide, sulfate, disulfide, sulfinamide, sulfonamide, thioester, aryl, silane, siloxane, heterocycles, thiocarbonate, thiocarbamate, and phosphonamide.

Another example is amination of the surface of a medical device. If the surface of a core material has hydroxy groups, the medical device may be placed in a bath of an inert solvent, such as tetrahydrofuran, and tresyl chloride. The hydroxy groups on the surface are then tresylated. Once tresylated, the surface may be aminated in a water solution of ethylene diamine, which results in bonding the group -NH-CH₂-CH₂-NH₂ to the carbon atom thereon.

Alternatively, for example, a contact lens made from a hydrogel, can be dipped into or sprayed with a solution containing a diaziridine compound, which is subsequently attached covalently to the surface of the contact lens via a thermal process, so as to functionalize the contact lens. Such functionalized lenses can be used in covalently attaching of a layer of a receptor.

Alternatively, for example, a contact lens made from a hydrogel, can be dipped into or sprayed with a solution containing a diaziridine compound, which is subsequently attached covalently to the surface of the contact lens via a thermal process, so as to functionalize the contact lens.

A receptor-containing coating on a contact lens can also be a coating comprising an LbL coating that is not covalently attached to the contact lens and a layer of a receptor which are covalently attached to the LbL coating through the reactive sites of the LbL coating. Such coating can be made, for example, by first applying an LbL coating to a preformed contact lens according to one of the above-described coating methods using at least one polyionic material having functional groups which will be served as reactive sites and then by covalently attaching a layer of a receptor to some of those reactive sites.

A receptor can be bound covalently to the LbL coating. This may be either a direct reaction or, preferably, a reaction in which a coupling agent is used. For example, a direct reaction may be accomplished by the use of a reagent of reaction that activates a group in the LbL coating or the receptor making it reactive with a functional group on the receptor or LbL coating, respectively, without the incorporation of a coupling agent. For example, one or more amine groups on a protein (e.g., receptor protein or antibody) may be reacted directly with isothiocyanate, acyl azide, N-hydroxysuccinimide ester, sulfonyl chloride, an aldehyde, glyoxal epoxide, 25 carbonate, aryl halide, imido ester, or an anhydride group in the LbL coating.

Alternatively, coupling agents may be used. Coupling agents useful for coupling a receptor to the LbL coating of a contact lens include, without limitation, N. N'-carbonyldiimidazole, carbodiimides such as 1-ethyl-3-(3-dimethylaminopropyl)carbodiimide ('EDC"), dicyclohexyl carbodiimide, 1-cylcohexyl-3-(2-morpholinoethyl)carbodiimide, diisopropyl carbodiimide, or mixtures thereof. The carbodiimides also may be used with N-hydroxysuccinimide or N-hydroxysulfosuccinimide to form esters that can react with amines to form amides.

Amino groups also may be coupled to the LbL coating by the formation of Schiff bases that can be reduced with agents such as sodium cyanoborohydride and the like to form hydrolytically stable amine links. Coupling agents useful for this purpose include, without limitation, N- hydroxysuccinimide esters, such as dithiobis(succinimidylpropionate), 3, 3'-dithiobis(sulfosuccinimidylpropionate), disuccinimidyl suberate, bis(sulfosuccinimidyl) suberate, disuccinimidyl tartarate and the like, imidoesters, including, without limitation, dimethyl adipimate, difluorobenzene derivatives, including without limitation 1,5-difluoro-2, 4 dinitrobenzene, bromofunctional aldehydes, including without limitation gluteraldehyde, and his epoxides, including without limitation 1,4- butanediol diglycidyl ether. One ordinarily skilled in the art will recognize that any number of other coupling agents may be used depending on the functional groups present in the LbL coating.

A receptor can be encapsulated in a vesicle with surfaces charges, which in turn is used to prepare an LbL coating on a contact lens, as described in a co-pending US patent application No. 60/364,192, filed on March 13, 2002, entitled "Materials Containing Multiple Layers of Vesicles". In accordance with the present invention, vesicles include polymerized liposomes, polymerized micelles, and nanocapsules or miorocapsules each having a multilayered shell of polyelectrolytes. A person skilled in the art will know how to prepare vesicles with receptor encapsulated therein.

In accordance with the invention, a contact lens of the invention comprises molecular imprints for the analyte of interest. For example, a contact lens can be made from a polymerizable composition comprising an analyte of interest. After curing the polymerizable composition, the analyte of interest can be extracted to provide molecular imprints for the analyte of interest in the contact lens.

A method for collecting an analyte of interest in a tear fluid comprises the steps of: providing a contact lens capable of binding the analyte of interest; wearing the contact lens on an eye of an individual for a period of time so that an amount of the analyte of interest is absorbed by the contact lens; and removing the contact lens containing the amount of the analyte of interest from the eye.

For collecting an analyte of interest in a tear fluid, a contact lens of the invention is wore preferably for at least 30 minutes, more preferably for at least 2 hours, even more preferably for at least 4 hours, much more preferably for at least 6 hours.

In accordance with a preferred embodiment of the present invention, a contact lens of the invention can be used to collect one or more analytes of interest, since a plurality of receptors, each for a specific analyte of interest can be incorporated into the contact lens of the invention.

A kit for collecting an analyte of interest in a body fluid comprises: a contact lens for collecting an analyte of interest in a tear fluid, wherein said contact lens is capable of binding the analyte of interest and has surface charges that can impart to the contact lens an increased adsorption of the analyte of interest, a coating comprising a receptor which can bind specifically the analyte of interest, or molecular imprints for the analyte of interest; and an instruction.

A method for assaying an analyte of interest in a body fluid comprises the steps of: providing a contact lens capable of binding the analyte of interest; wearing the contact lens on an eye of an individual for a period of time, preferably 30 minutes or longer, more preferably 2 hour or longer, so that an amount of the analyte of interest is absorbed by the contact lens; removing the contact lens containing the amount of the analyte of interest from the eye; determining the presence or the amount of the analyte of interest.

It is well known to a skilled artisan that assay of an analyte of interest can be carried out with the help of a testing agent composition which specifically reacts or interacts with the analyte of interest, leading to formation of a detectable signal. A detectable signal, for example, can be radio signals (i.e., radioactive isotopes), electrical signals, or optical signals. Exemplary electrical signals are electrical potentials and currents. Optical signals refers to changes in

the optical properties, including, but not limited to, a color formation, a change in color, fluorescence, luminescence, chemiluminescence, changes in fluorescence or luminescence intensity, changes in fluorescence or luminescence lifetimes, fluorescent anisotropy or polarization, a spectral shift of the emission spectrum, time-resolved anisotropy decay, and the like.

Any known suitable assays can be used in the present invention. Exemplary assays include, without limitation, radioimmunoassay (RIA), enzyme immunoassay (EIA), immunofluorescence assay (IFA), enzyme-linked immunosorbent assay (ELISA), assays based on Trinder reaction, electrochemical assay, and the like.

Assays can be performed directly on a fraction or all of a contact lens of the invention. Alternatively, assays can be performed in a recovered tear sample containing the analyte of interest from the contact lens according to any known suitable method (for example, such as vacuum, squeezing, or the like).

A kit for assaying an analyte of interest in a body fluid comprises: a contact lens for collecting an analyte of interest in a tear fluid, wherein said contact lens is capable of binding the analyte of interest and has molecular imprints for the analyte of interest; and a testing agent composition which specifically reacts or interacts with the analyte of interest to form a detectable signal.

Methods and kits of the invention are useful for diagnostic purposes, for example, to test for pregnancy (to detect β-HCG), to assess blood chemistry (electrolytes, Ca₂PO₄, magnesium, bilirubin, alkaline phosphatase, lactate dehydrogenase, alanine aminotransferase, etc.), to detect infection (*e*.*g*., by detecting components of viruses such as CMV, EBV, hepatitis, and HIV, or bacteria, such as *Staphlococcus*, *Streptococcus*, etc.), and to detect anything in a tear fluid that correlates to the physiological or diseased states of a patient. They also are useful for monitoring blood levels of test compounds during the course of assessing the compounds for use as potential therapeutics.

By using a contact lens, preferably a daily disposable contact lens, of the invention, a contact lens wearer can monitor their health via their contact lens. After a days wear of the contact lens they could remove it and perform one or more assays to determine the presence or the amount of one or more analytes of interest. For example, it could allow people to monitor, in particular but not solely, ovulation, pregnancy, contraception, menopause or sexual dysfunction in a more convenient and discreet manner (e.g., at home), without the inconvenience or embarrassment of using first morning urine or urine strip tests.

The previous disclosure will enable one having ordinary skill in the art to practice the invention. In order to better enable the reader to understand specific embodiments and the advantages thereof, reference to the following examples is suggested.

### Example 1

A DAILIES® contact lens, made of a modified polyvinylalcohol (Nelfilcon A), was placed In one of the wells of a 24-well plate. Each well contains 1 ml of a progesterone solution (70 nmol/L of progesterone which is within the range of a physiological concentration). The lens was soaked in the progesterone for 12 hours and then washed thoroughly and extracted via vacuum for any progesterone. A commercially available immunoassay kit (Bioclone) was used to determine how much progesterone had been absorbed into the lens. The results indicated that the contact lens had taken up approximately 3% of total amount of progesterone in the soaking solution.

### Example 2

**Polyacrylic acid (PAA) solution:** A PAA solution (0.001 M, pH 2.5) is prepared from a polyacrylic acid having a molecular weight of about 90,000, from Polyscience, Inc. The PAA concentration is calculated based on the repeating unit in PAA. **Poly(allylamine hydrochloride) (PAH) solution:** A PAH (0.001 M, pH ∼4.3) is prepared from a PAH having a molecular weight of about 60,000, from Aldrich. The PAH concentration is calculated based on the repeating unit in PAH.
**Coating A (PAA/PAH/PAA/PAH/PAA/PAH/PAA/PAH/PAA/PAH):** An LbL coating having 4 bilayers of PAA/PAH is formed on a DAILIES® contact lens, made of a modified polyvinylalcohol material, Nelfilcon A, (CIBA Vision). The contact lens is dipped with the help of a Zeiss coater in a PAA solution (0.001 M, pH 2.5) for 30 minutes to form the Innermost layer of the coating on the lens and then rinsed with water by dipping with the help of a Zeiss coater in water for I minute. The lens with the innermost layer of PAA is then dipped with the help of a Zeiss coater in a PAH solution (0.001 M, pH ∼4.3) for 5 minutes, rinsed with water by dipping with the help of a Zeiss coater in water, dipped with the help of a Zeiss coater in the PAA solution (0.001 M, pH 2.5) for 5 minutes, and then rinsed by dipping with the help of a Zeiss coater in water. The steps of alternatively dipping with the help of a Zeiss coater in the PAA solution for 5 minutes and in PAH solution for 5 minutes are repeated for a number of time to build up 4 bilayers (i.e., PAA/PAH/PAA/PAH/PAA/PAH/PAA/PAH) with a capping layer of PAH on the lens. The capping layer of coating A is a PAH layer.
**Coating B (PAA/PAH/PAA/PAH/PAA/PAH/PAA/PAH/PAA/PAH/PAA):** An LbL coating having 4 bilayers of PAA/PAH and a capping layer of PAA is formed on a DAILIES® contact lens, made of a modified polyvinylalcohol material, Nelfilcon A, (CIBA Vision). The contact lens is dipped with the help of a Zeiss coater in a PAA solution (0.001 M, pH 2.5) for 30 minutes to form the innermost layer of the coating on the lens and then rinsed with water by dipping with the help of a Zeiss coater in water for I minute. The lens with the innermost layer of PAA is then dipped with the help of a Zeiss coater in a PAH solution (0.001 M, pH ∼4.3) for 5 minutes, rinsed with water by dipping with the help of a Zeiss coater in water, dipped with the help of a Zeiss coater in the PAA solution (0.001 M, pH 2.5) for 5 minutes, and then rinsed by dipping with the help of a Zeiss coater in water. The steps of dipping with the help of a Zeiss coater in the PAH solution for 5 minutes followed by dipping with the help of a Zeiss coater in the PAA solution for 5 minutes are repeated for 3 additional times to build up 4.5 bilayers (i.e., PAA/PAH/PAA/PAH/PAA/PAH/PAA/PAH/PAA) on the lens. The capping layer of coating B is a PAA layer.

Contact lenses without LbL coating, with coating A and with coating B are placed in wells of a 24-well plate (one lens in one well). Each well contains 1 ml of an enzyme-labeled LH solution. Each contact lens is soaked in an enzyme-labeled LH solution overnight. The tested concentrations of enzyme-labeled luteinzing hormone is ranged from 0.1 µg/mL to 0.5 mg/ml. After soaking, the lenses are washed thoroughly and assayed for the presence of enzyme-labeled LH. Results show that the lenses with coating B (with a capping layer of PAA ) absorb LH significantly higher than the lenses without coating or the lenses with coating A (with a capping layer of PAH).

### Example 3

A DAILIES® contact lens, made of a modified polyvinylalcohol material, Nelfilcon A, (CIBA Vision) is coated to form an LBL coating (PAA/PAH/PAA/PAH/PAA/PAH/PAA/PAH/PAA) as described in Example 2 (Coating B). The LbL coating provided a surface with free COOH groups to which the amine groups of an LH monoclonal antibody are covalently attached by using EDC/s-NHS coupling as follows.

LH antibody - horseradish peroxidase (HRP) conjugate is prepared by using EZ-Link Maleimide Activated HRP kit from Pierce according to the procedure recommended by the supplier. The obtained LH antibody - HRP conjugate is used to prepare a conjugate solution (0.16 mg/ml in water).
An EDC/s-NHS solution is prepared by dissolving 100 mg of EDC (1-Ethyl-3-(3-dimethylaminopropyl)) and 220 mg of s-NHS (N-Hydroxysulfosuccinimide) in 10 ml water. 5 ml of the above-prepared EDC/s-NHS solution is mixed with 5 ml of the above-prepared LH antibody-HRP conjugate solution and the pH of the solution is adjusted to about 9.0 to obtain a crosslinking solution.
Each of contact lenses with coating B is placed in a well of a 24-well plate. Each well contains 1 ml of the crosslinking solution. Each lens is soaked in the covalently attachment solution at 4°C overnight. After overnight soaking, each lens is rinsed 4 times with PBS (1 hour per rinse). After final rinse, each lens is transferred to one of the wells of a clean 24-well plate (1 lens per well) and soaked in OPD substrate solution (o- Phenylenediamine). At the reaction endpoint, 100 µL of aliquots from each well are placed in a 96-well plate and the absorption at 450 nm is determined to calculate the amount of LH antibody-HRP conjugate covalently attached to the lens. It is found that in average 80 ng of LH antibody-HRP conjugate is covalently attached to each DAILIES lens with coating B. Such amount of LH antibody-HRP conjugate is shown to be sufficient to attract LH from the tear film enabling direct measurement of the presence of LH from the lens.

## Claims

1. A contact lens for collecting an analyte of interest in a tear fluid, comprising molecular imprints for the analyte of interest.

2. A contact lens of claim 1, wherein the contact lens further has surface charges which are introduced by: (1) preparing the contact lens from a composition comprising a positively or negatively charged monomer or macromer; (2) altering the chemical nature of chemical groups on the surface of the contact lens; (3) applying an LbL coating composed of at least one layer of a polyionic material onto the contact lens; or (4) combinations of (1), (2) and (3).

3. A contact lens of claim 2, wherein the contact lens has surface charges which are introduced by applying an LbL coating composed of at least one layer of a polyionic material onto the contact lens.

4. A contact lens of any one of claims 1 to 3, wherein the contact lens further has a coating comprising a receptor which binds specifically the analyte of interest.

5. A contact lens of claim 4, wherein the receptor is selected from the group consisting of antibodies, lectins, hormone receptors, drug receptors, enzymes, aptamers, nucleic acids, nucleic acid analogs, and fragments thereof.

6. A contact lens of any one of claims 1 to 5, wherein the contact lens further has a core material that is prepared from a composition containing a receptor which binds specifically the analyte of interest.

7. A contact lens of any one of claims 1 to 6, wherein the contact lens is a soft contact lens.

8. A contact lens of claim 7, wherein the soft contact lens is a daily disposable hydrogel lens.

9. A method for collecting an analyte of interest in a tear fluid, comprising the steps of:
providing a contact lens capable of binding the analyte of interest,wherein the contact lens has molecular imprints for the analyte of interest; wearing the contact lens on an eye of an individual for a period of time sufficient to absorb an amount of the analyte of interest; and removing the contact lens containing the amount of the analyte of interest from the eye.

10. A method for assaying an analyte of interest in a tear fluid, comprising the steps of:
providing a contact lens capable of binding the analyte of interest, wherein the contact lens has molecular imprints for the analyte of interest; wearing the contact lens on an eye of an individual for a period of time sufficient long to absorb an amount of the analyte of interest; removing the contact lens containing the amount of the analyte of interest from the eye; determining the presence or the amount of the analyte of interest.

11. A kit for collecting an analyte of interest in a tear fluid, comprising: a contact lens according to any one of claims 1 to 6; and an instruction.

12. A kit for assaying an analyte of interest in a tear fluid, comprising: a contact lens according to any one of claims 1 to 7; and a testing agent composition which specifically reacts or interacts with the analyte of interest to form a detectable signal.

## Patentansprüche

1. Kontaktlinse zum Auffangen von interessierenden Analyten in einer Tränenflüssigkeit, umfassend molekulare Abdrücke für den interessierenden Analyten.

2. Kontaktlinse gemäss Anspruch 1, wobei die Kontaktlinse ferner Oberflächenladungen hat die eingeführt werden durch: (1) Herstellen der Kontaktlinse aus einer Zusammensetzung umfassend ein positiv oder negativ geladenes Monomer oder Makromer; (2) Verändern der chemischen Natur von chemischen Gruppen auf der Oberfläche der Kontaktlinse; (3) Aufbringen einer Schicht-für-Schicht Beschichtung, bestehend aus mindestens einer Schicht eines polyionischen Materials, auf die Kontaktlinse; oder (4) Kombinationen aus (1), (2) und (3).

3. Kontaktlinse gemäss Anspruch 2, wobei die Kontaktlinse Oberflächenladungen hat die eingeführt werden durch Aufbringen einer Schicht-für-Schicht Beschichtung bestehend aus mindestens einer Schicht eines polyionischen Materials auf die Kontaktlinse.

4. Kontaktlinse gemäss einem der Ansprüche 1 bis 3, wobei die Kontaktlinse ferner eine Beschichtung aufweist, die einen Rezeptor umfasst, der spezifisch den interessierenden Analyten bindet.

5. Kontaktlinse gemäss Anspruch 4, wobei der Rezeptor ausgewählt ist aus der Gruppe bestehend aus Antikörpern, Lectinen, Hormonrezeptoren, Wirkstoffrezeptoren, Enzymen, Aptameren, Nukleinsäuren, Nukleinsäure-Analogen, und Teilen davon.

6. Kontaktlinse gemäss einem der Ansprüche 1 bis 5, wobei die Kontaktlinse ferner ein Kernmaterial umfasst, das aus einer Zusammensetzung hergestellt wird, die einen Rezeptor, der spezifisch den interessierenden Analyten bindet, umfasst.

7. Kontaktlinse gemäss einem der Ansprüche 1 bis 6, wobei die Kontaktlinse eine weiche Kontaktlinse ist.

8. Kontaktlinse gemäss Anspruch 7, wobei die weiche Kontaktlinse eine Einweg, Eintages Hydrogel-Linse ist.

9. Verfahren zum Auffangen eines interessierenden Analyten in einer Tränenflüssigkeit, umfassend die Schritte: Bereitstellen einer Kontaktlinse die den interessierenden Analyten binden kann, wobei die Kontaktlinse molekulare Abdrücke für den interessierenden Analyten hat; Tragen der Kontaktlinse auf dem Auge eines Individuums für einen Zeitraum der ausreichend ist eine Menge des interessierenden Analyten zu absorbieren; und Entfernen der Kontaktlinse eine Menge des interessierenden Analyten enthält vom Auge.

10. Verfahren zur Bestimmung eines interessierenden Analyten in einer Tränenflüssigkeit, umfassend die Schritte: Bereitstellen einer Kontaktlinse die den interessierenden Analyten binden kann, wobei die Kontaktlinse molekulare Abdrücke für den interessierenden Analyten hat; Tragen der Kontaktlinse auf dem Auge eines Individuums für einen Zeitraum der ausreichend ist eine Menge des interessierenden Analyten zu absorbieren; und Entfernen der Kontaktlinse eine Menge des interessierenden Analyten enthält vom Auge; Feststellen der Anwesenheit oder der Menge des interessierenden Analyten.

11. Kit zum Auffangen interessierender Analyten in einer Tränenflüssigkeit, umfassend:
Kontaktlinse gemäss einem der Ansprüche 1 bis 6; und eine Anleitung.

12. Kit zum Auffangen interessierender Analyten in einer Tränenflüssigkeit, umfassend:
Kontaktlinse gemäss einem der Ansprüche 1 bis 7; und eine Prüfmittelzusammensetzung welche spezifisch mit dem interessierenden Analyten reagiert oder wechselwirkt um ein nachweisbares Signal zu erzeugen.

## Revendications

1. Lentille de contact permettant de recueillir un analyte d'intérêt dans un liquide lacrymal, comprenant des empreintes moléculaires pour l'analyte d'intérêt.

2. Lentille de contact selon la revendication 1, laquelle lentille de contact a en outre des charges superficielles qui sont introduites en : (1) préparant la lentille de contact à partir d'une composition comprenant un monomère ou macromère chargé positivement ou négativement ; (2) modifiant la nature chimique de groupes chimiques à la surface de la lentille de contact ; (3) déposant un revêtement LbL composé d'au moins une couche d'un matériau polyionique sur la lentille de contact ; ou (4) des combinaisons de (1), (2) et (3).

3. Lentille de contact selon la revendication 2, laquelle lentille de contact a des charges superficielles qui sont introduites en déposant un revêtement LbL composé d'au moins une couche d'un matériau polyionique sur la lentille de contact.

4. Lentille de contact selon l'une quelconque des revendications 1 à 3, laquelle lentille de contact a en outre un revêtement comprenant un récepteur qui lie de manière spécifique l'analyte d'intérêt.

5. Lentille de contact selon la revendication 4, dans laquelle le récepteur est choisi dans le groupe constitué par les anticorps, les lectines, les récepteurs hormonaux, les récepteurs de médicaments, les enzymes, les aptamères, les acides nucléiques, les analogues d'acides nucléiques, et leurs fragments.

6. Lentille de contact selon l'une quelconque des revendications 1 à 5, laquelle lentille de contact a en outre un matériau central qui est préparé à partir d'une composition contenant un récepteur qui lie de manière spécifique l'analyte d'intérêt.

7. Lentille de contact selon l'une quelconque des revendications 1 à 6, laquelle lentille de contact est une lentille de contact souple.

8. Lentille de contact selon la revendication 7, laquelle lentille de contact souple est une lentille hydrogel à remplacement quotidien.

9. Procédé permettant de recueillir un analyte d'intérêt dans un liquide lacrymal, comprenant les étapes consistant à : obtenir une lentille de contact capable de lier l'analyte d'intérêt, la lentille de contact ayant des empreintes moléculaires pour l'analyte d'intérêt ; placer la lentille de contact sur l'oeil d'un individu pendant une période de temps suffisante pour absorber une quantité de l'analyte d'intérêt ; et retirer de l'oeil la lentille de contact contenant la quantité de l'analyte d'intérêt.

10. Procédé permettant de doser un analyte d'intérêt dans un liquide lacrymal, comprenant les étapes consistant à : obtenir une lentille de contact capable de lier l'analyte d'intérêt, la lentille de contact ayant des empreintes moléculaires pour l'analyte d'intérêt ; placer la lentille de contact sur l'oeil d'un individu pendant une période de temps suffisante pour absorber une quantité de l'analyte d'intérêt ; retirer de l'oeil la lentille de contact contenant la quantité de l'analyte d'intérêt ; déterminer la présence ou la quantité de l'analyte d'intérêt.

11. Trousse permettant de recueillir un analyte d'intérêt dans un liquide lacrymal, comprenant : une lentille de contact selon l'une quelconque des revendications 1 à 6 ; et un mode d'emploi.

12. Trousse permettant de doser un analyte d'intérêt dans un liquide lacrymal, comprenant : une lentille de contact selon l'une quelconque des revendications 1 à 7 ; et une composition d'agents réactifs qui réagit ou interagit de manière spécifique avec l'analyte d'intérêt pour former un signal détectable.
